Europäisches Patentamt

European Patent Office

Office européen des brevets

(11) Publication number: **0 213 415**
**B1**

(12) **EUROPEAN PATENT SPECIFICATION**

(45) Date of publication of patent specification: **02.01.91**

(21) Application number: **86110680.5**

(22) Date of filing: **01.08.86**

(51) Int. Cl.⁵: **C 08 B 15/06, A 61 L 15/00, D 21 C 9/00, D 06 M 13/322**

(54) Method for preparing modified cellulosic fibers.

(30) Priority: **02.08.85 US 763301**

(43) Date of publication of application:
**11.03.87 Bulletin 87/11**

(45) Publication of the grant of the patent:
**02.01.91 Bulletin 91/01**

(84) Designated Contracting States:
**BE DE FR GB**

(56) References cited:
**WO-A-85/03509**
**FR-A-2 310 356**
**US-A-3 029 232**

**CHEMICAL ABSTRACTS, vol. 86, no. 20, 16th May 1977, page 75, abstract no. 141502c, Columbus, Ohio, US; M. SOLARZ: "Modification of cellulose fibers with N,N1-methylenebisacrylamide", & PRZEGL. WLOK. 1976, 30(11-12), 546-9**

(73) Proprietor: **SCOTT PAPER COMPANY**
**Industrial Highway Tinicum Island Road Tinicum Township**
**Delaware County, PA 19113 (US)**

(72) Inventor: **Box, Larry**
**90 Andrien Road**
**Glen Mills Pennsylvania 19342 (US)**

(74) Representative: **Wuesthoff, Franz, Dr.-Ing. et al**
**Wuesthoff & Wuesthoff Patent- und Rechtsanwälte Schweigerstrasse 2**
**D-8000 München 90 (DE)**

Courier Press, Leamington Spa, England.

**Description**

This invention relates to the use of a special method for modifying of cellulosic materials to increase bulk and absorbency.

The invention starts from the process defined in applicants own WO 85/03509, which has not been prepublished and according to which a method for preparing of modified cellulosic fibres is proposed. In said process, the fibre material is reacted with N,N'-methylene-bisacrylamide in an aqueous alkaline medium having a pH between 8.5 and 13.5 and having a consistency of at least 10%, excluding air and maintaining the temperature of the aqueous medium below its boiling point.

US—A—3,029,232 describes a method for avoiding hydrolysis in the preparation of acrylamide derivatives of cellulose by reacting alpha-cellulose from wood pulp with NaOH, water, N,N'-methylene-bisacrylamide, sodium thiocyanate and hydroquinone; the consistency being 5%.

It is the object of the subject invention to prepare products having the properties of high water absorption and bulk.

For solving this problem fines or wood pulp fibers derived from a mechanical pulping process are treated with N,N'-methylene-bisacrylamide in an aqueous alkaline medium having a pH between 8.5 and 13.5 and having a consistency of at least 10%, excluding air and maintaining the temperature of the aqueous medium below its boiling point. It is preferred that the fibres are in the form of a slurry having a consistency between 20 and 45% or in the form of a batt or web having a consistency greater than 45%.

In accordance with the method of the present invention, the reaction is carried out in an aqueous alkaline medium having a pH between 8.5 and 13.5 and preferably between 11 and 13, using either an aqueous slurry or mixture of cellulosic fibers or by applying the N,N'-methylenebisacrylamide in solution to a batt or web of cellulosic fibers of fines. The amount of N,N'-methylenebisacrylamide to be dissolved in the aqueous alkaline medium with the cellulosic fibrous material should be sufficient to impart the desired increase in bulk and absorbency. Above about 25% N,N'-methylenebisacrylamide by dry weight of the fibers there is no additional effect on the fibers. Insufficient N,N'-methylenebisacrylamide generally below 0.2%, will produce no perceptible change in the bulk or total water absorption of a web of the fibers, although any measurable amount of N,N'-methylenebisacrylamide produces a measurable increase in absorbancy i.e. the rate of absorption. In practice using sufficient N,N'-methylenebisacrylamide to cause from about 0.5 to 2% by dry weight of the fibers to react with the fibers produces a level of modification sufficient for commercial applications.

In accordance with an alternative embodiment of the present invention, when it is desired to carry out the reaction at very high consistencies, for example above 50%, the aqueous alkaline medium (in which the N,N'-methylenebisacrylamide is soluble) may be incorporated into a water miscible organic solvent such as acetone. This combination provides a greater volume in which to slurry or immerse the fibers and with which to protect the fibers from air. Therefore, as used herein the term "aqueous alkaline medium" comprehends mixtures of water with water-miscible organic solvents which do not interfere with the reaction between N,N'-methylenebisacrylamide and the cellulosic fibers.

The modified fibers of this invention may be used in combination with conventional papermaking fibers to produce webs which exhibit increased bulk and absorbency. The modified fibers of the present invention improve the bulk and absorbency of the base web in direct proportion to the percentage of modified fiber in the blend. Alternatively, blending can permit a reduction in basis weight while retaining bulk and absorbancy. By way of illustration, in a web containing 30% modified fiber, basis weight was reduced by 25% without loss of bulk and absorbancy as compared with a web without modified fibers of the present invention. Another utility of the fibers of the invention is as a replacement for any of the known super absorbent fibers such as the "super slurper" fibers for use in a variety of absorbent products such as diapers, sanitary napkins, hospital dressings and the like. Unlike the typical super-absorbent fibers which characteristically have a gelatinous or "slimy" feel when wet, the fibers made in accordance with the present invention retain their wood pulp-like feel to the touch. A further advantage of the present invention is that the modified fibers exhibit wet resilence. That is to say, the fibers retain their bulk when wet which is important in many absorbent products, such as diapers, where the shape and volume of the product when wet plays an important part in the function of the product e.g. in retaining fit and wicking. Previous inventors have attempted to achieve these same properties, but found it necessary to employ rather elaborate and severe chemical conditions to achieve only some of these properties. For example, US—A—4,248,595, discloses a method to produce "swellable carboxyalkylcelluloses" from a two-part chemical system comprising first a carboxyalkyl etherifying agent and second a crosslinking agent in an aqueous alkaline medium. Furthermore, a very much larger proportion of the etherifying agent and alkali based on the weight of fiber is required by said process relative to N,N'-methylenebisacrylamide, which is employed solely to crosslink the etherifying agent. In the present invention no such etherifying agent is employed, a much lower alkali concentration is required and the reaction chemistry of N,N'-methylenebisacrylamide resembles that of the carbamoylethylation class of reactions as opposed to crosslinking as described said prior art.

The present inventor is unaware of any disclosure in the prior art which would suggest such results from N,N'-methylenebisacrylamide. Indeed, several other amides and acrylates were tried but did not produce clear improvements in absorbancy and bulk. These compounds include acrylamide, N-methylolacrylamide, bisacrylamide glyoxal, N-isopropylacrylamide, N,N-dimethylamino ethyl methacrylate, triallylcyanurate, glycidyl acrylate and acrylic acid.

The remarkable results attained with N,N'-methylenebisacrylamide in accordance with the present invention are especially surprising in view of the prior art. The alkali concentrations employed in the present invention are relatively low. In such alkali media of relatively low concentration, the base catalyzed reaction of cellulose with acrylamide has long been believed to produce carbamoylethylcellulose. US—A—2,338,681 does not suggest that carbamoylethylcellulose provides greater bulk and absorbency to webs. Indeed, the pertinent prior art suggest that such forms of cellulose are soluble in water when sufficiently modified with acrylamide. In the above mentioned US—A—3,029,232 is described in Example 8 that paper made from cellulose treated with N,N-propylacrylamide is stronger than from untreated pulp. However, they reported no change in absorbancy of the fibers. The fibers obtained according to the inventive process, in contrast, form weaker webs having greater absorbency and bulk. It is to be concluded, therefore, that the reaction product of the present invention has a chemical composition different from or comprises something in addition to carbamoylethylated cellulose as understood by the prior art.

Treatment of wood pulp fibers treated in accordance with the present invention results in extended hydrophilicity, increased brightness and receptivity to ink, and in fibers which are more readily debonded thus resulting in bulkier, softer paper than the same fiber in the untreated form. There is at the same time no perceptible change in the structural appearance of the fibers.

These improved properties are retained by the fibers when subjected to typical stress to which pulp fibers are subjected, for example, boiling water, moderately strong acid and alkali, and bleaching chemicals. Bleaching with chlorine, chlorine dioxide, hydrogen peroxide, ozone and combinations of such bleaching steps will not undo the fiber modification. This persistence of the structural integrity of the fibers (as well as the improved properties) further distinguishes the fibers resulting from the process of the present invention from those carbamoylethylated cellulose fibers of the prior art which are soluble in water (US—A—3,029,232, Column 5, lines 14—15).

In one embodiment of the present invention, the N,N'-methylenebisacrylamide reagent is combined with the sodium hydroxide used for the second extraction stage during a bleaching sequence such as chlorine-alkali extraction—hypochlorite-alkali extraction—chlorine dioxide or chlorine-alkali extraction—chlorine dioxide-alkali extraction—chlorine dioxide. The temperature e.g. 60°C, and duration, typically one hour, of such a stage are sufficient for the modification reaction to be completed. The final bleach stage (chlorine dioxide) serves as a neutralization and washing step.

The present invention provides overall improvement in the wood pulps produced by "high yield" or mechanical pulping processes, e.g. thermomechanical and refiner mechanical pulps, which are characterized in having larger hemicellulose and carbohydrate contents than chemical pulp. Since, as previously mentioned, the modification imparted to cellulosic fibers by N,N'-methylenebisacrylamide survives delignification treatments, the aforementioned treatment of "high yield" or mechanical pulps can be advantageously combined with a delignification step, either prior to or subsequent to application of the method of the present invention.

Mechanical pulps are desirable in that they are produced in high yield, but have found limited use in absorbent paper products due to their rigid structure. In the past, attempts have been made to produce fibers which have enhanced flexibility compared to groundwood, refiner mechanical pulp, thermomechanical pulp, chemirefiner and chemi-thermomechanical pulp through delignification. Unfortunately, total or partial delignification produces pulps with reduced bulk. The latter phenomenon is disadvantageous when the end-use of the fiber is in absorbent products.

In accordance with the present invention mechanical fibers can be made flexible while maintaining or improving their bulk characteristics. These fibers are subjected to, for example, ozonization whereby at least partial delignification is achieved, resulting in low bulk high-bonding fibers, which upon subsequent treatment in accordance with the present invention results in a pulp with high brightness, bulk and flexibility. Alternatively, the method of the present invention can be applied prior to the delignification stage. In this connection, it is to be noted that fibers treated in accordance with the present invention exhibit sharp reductions in bleach chemical demand.

A further advantage of the present invention is that when short cellulosic fibers, hereinafter called fines, are modified in accordance with the present invention, they become non-bonding and dispersible. This feature has significant economic implications. In particular, it permits the use of pulp furnishes containing a high proportion of fines without the normal difficulties. Wood fines, an assortment of particulate wood products which pass through a 75 nm opening, exhibit rather noticeable adhesive properties uncommon to regular wood fibers. Consequently, when isolated and dried they form a dense agglomerated structure which resists being dispersed in water. In this agglomerated state, fines interfere with both the manufacture of absorbent papers and their product qualities. When fines are treated in accordance with the present invention, they become soft and dispersible in water after drying. By way of illustration, a stone ground wood pulp containing 23% fines, when formed into a mat from an aqueous dispersion, dried into a rough textured non-dispersible mass. When the same pulp was treated in accordance with the method of the present invention with 2.5% N,N'-methylenebisacrylamide based on dry pulp weight, neutralized and dried, the treated fines were found to be soft and dispersible.

It can be appreciated by one of ordinary skill in the art to which the present invention pertains that a

3

large number of variations may be effected in reacting the cellulosic fibrous material with N,N'-methylenebisacrylamide in accordance with the reaction procedures described above. The following examples will more fully illustrate the embodiments of this invention. "Wet pick-up" is expressed as a percent by weight of the dry fibers to which the solution is applied. The abbreviation "TWA" stands for "total water absorbed" and is determined on a gram for gram basis, e.g. a TWA of 2 means 2 grams of water were absorbed for each gram of fiber. The ratio of bulk to basis weight is a means of comparing the bulk of webs of different basis weight. A larger ratio indicates greater bulkiness (lesser density) and is regarded as an improvement in sanitary tissue. This ratio and TWA are the principal criteria for measuring the improvements produced in accordance with the present invention. "Bulk" is expressed as the height in millimeters of a stack of 24 sheets.

Tensile measurements were obtained on a Thwing Albert Tensile Tester in accordance with TAPPI Standard Number T 456m-49. Tensile was measured cross direction (CD) and machine direction (MD) for a dry strip.

Example 1

A roll of paper having a basis weight of 30 g/m² and made from northern softwood kraft pulp was saturated by means of a gravure apparatus to the extent of 74% wet pick-up with a solution comprising 6% by weight N,N'-methylenebisacrylamide and 5% potassium hydroxide. The resulting impregnated web had a consistency of 58%. After a 14-day reaction period at room temperature in sealed plastic wrap, specimens were withdrawn for evaluation. The results are shown in Table 1. Sample A is a dry sheet made from the same lot of northern softwood kraft pulp as was treated in this Example. Sample B represents Sample A after refining in a Valley beater to increase its breaking length. Sample C represents the modified pulp described in this Example. Sample D is a sheet made from a dispersion of the sheets of Samples B and C mixed in equal proportions, formed into a web and dried.

Example 2

Modified fiber in accordance with the present invention was produced by feeding northern softwood kraft pulp saturated to the extent of 330% wet pick-up with a solution comprising 2.9% N,N'-methylenebisacrylamide and 1.4% potassium hydroxide in a steam-jacketed high consistency continuous refiner mixing device heated to 80°C where it resided for two minutes. The reacted pulp was collected at a consistency of 28%, diluted and acidified with phosphoric acid to pH 7 and transferred to a paper machine where the modified pulp was blended with untreated northern softwood kraft in the proportion of 50% by dry weight to all the fibers to produce towel weight webs of 41 g/m². The results are presented in Table 2.

TABLE 1

| | | % MBA[3] in composite | Basis wgt[4] g/m² | Breaking[1] length m | TWA[2] g/g | Bulk basis weight[5] lbs (mils/———) 2880 sq.ft | kg mm/——— m² |
|---|---|---|---|---|---|---|---|
| A. | Control | 0 | 44.0 | 2621 | 2.84 | (4.8) | 72 |
| B. | Control Refined | 0 | 39.0 | 9040 | 1.92 | (4.3) | 64.5 |
| C. | Modified Fiber | 1.93 | 41.0 | 945 | 5.40 | (7.1) | 106.5 |
| D. | B/C=1/1 Blend | 0.97 | 44.0 | 4114 | 3.72 | (5.2) | 78 |

[1] "Breaking length" is the estimated length at which the web would break under its own weight. It is derived from a measurement of the tensile strength of the web and related to its basis weight. While non-empirical, it is useful in comparing webs of different weights.

[2] TWA=total water absorbed

[3] MBA=N,N'-Methylenebisacrylamide

[4] Basis Weight=weight per unit of area of the web

[5] Bulk Basis Weight=ratio of bulk to basis weight

TABLE 2

| % Modified Fiber in Blend | 0 | 40 |
|---|---|---|
| Pulp Freeness (Canadian Standard) | 680 | 709 |
| Basis Weight g/m² | 40.0 | 41.0 |
| Assayed N,N'-methylenebisacrylamide % by weight of dry fiber in web | 0 | 0.66 |
| Physical Properties 24-ply bulk mm | 4.6 | 4.9 |
| total water absorbed g/g | 3.7 | 5.6 |
| Stretch (percent) measured in Machine Direction | 12.0 | 11.5 |
| Cross Direction | 1.7 | 2.7 |
| Dry Tensile measured in (ounce/inch) g/cm Machine Direction | (38.9) 434 | (19.5) 218 |
| Cross Direction | (30.3) 338 | (16.5) 184 |

Example 3

Sheets of dry lap pulp (southern softwood kraft weighing 800 g/m² on an air dry basis) were uniformly impregnated with a solution at a temperature of 50°C comprising 2.5% N,N'-methylenebisacrylamide and sufficient sodium hydroxide to achieve a pH of 11.5 using a gravure type applicator so as to provide a wet pick-up of 56% and a consistency of 64%. The impregnated pulp was rolled-up, enclosed in plastic film and allowed to react at room temperature for 30 days before quenching to pH 7.0 with a very dilute solution of aqueous phosphoric acid. The modified pulp contained 1.57% by weight N,N'-methylenebisacrylamide 56% of the amount applied based on nitrogen assays.

The modified pulp was blended with untreated, beaten southern softwood pulp in the proportion of 35% by dry weight to all the fibers to produce towel weight webs of approximately 50 and 40 g/m².

The degree of refining (REFINE) by means of a Valley beater, is represented in minutes. Refining of the samples with modified pulp was used to achieve a breaking length comparable to that of the control. As may be seen, a web of comparable TWA and bulk can be achieved at a much lower basis weight by incorporation of the modified pulp of the present invention.

## TABLE 3
### Control web at high basis weight

| REFINE min | basis weight g/m² | Bulk mm | Density g/cm³ | Bulk basis weight (mils/$\frac{lbs}{2880\ sq.ft}$) mm/$\frac{kg}{m^2}$ | Tensile in machine cross direction g/cm (oz/in) | g/cm (oz/in) | breaking length m | total water absorption g/g | Absorbency s |
|---|---|---|---|---|---|---|---|---|---|
| 5 | 53.2 | 5.28 | 0.516 | 99 (6.61) | 560 (53.40) | 420 (37.67) | 939.89 | 2.8 | 22.5 |

### Modified pulp substitution in web having lesser basis weight

| REFINE min | basis weight g/m² | Bulk mm | Density g/cm³ | Bulk basis weight (mils/$\frac{lbs}{2880\ sq.ft}$) mm/$\frac{kg}{m^2}$ | Tensile in machine cross direction g/cm (oz/in) | g/cm (oz/in) | breaking length m | total water absorption g/g | Absorbency s |
|---|---|---|---|---|---|---|---|---|---|
| 7 | 49.5 | 5.59 | 0.437 | 113 (7.54) | 358 (32.1) | 244 (21.9) | 595.67 | 3.7 | 6.9 |
| 15 | 43.1 | 4.64 | 0.531 | 108 (7.20) | 513 (46.0) | 326 (29.2) | 950.78 | 2.9 | 30.5 |

EP 0 213 415 B1

The absorbent products of the present invention can be used in a variety of applications where absorbency is desired. In particular they are useful in applications such as feminine hygiene products, catamenial devices, disposable diapers and non-wovens for hospital and surgical use.

**Claim**

A process for modifying fines or wood pulp fibers derived from a mechanical pulping process by reacting the fibers with N,N'-methylenebisacrylamide in an aqueous alkaline medium having a pH between 8.5 and 13.5 and having a consistency of at least 10%, excluding air and maintaining the temperature of the aqueous medium below its boiling point.

**Patentanspruch**

Verfahren zur Modifizierung von Holzschliff-Fasern oder deren Bruch aus dem mechanischen Faseraufschlämmen durch Umsetzung der Fasern mit N,N'-Methylenbisacrylamid in einem wäßrigalkalischen Medium mit einem pH-Wert von 8,5 bis 13,5 und einer Konsistenz von zumindest 10% einschließlich Luft, wobei die Temperatur des wäßrigen Mediums unter dem Siedepunkt gehalten wird.

**Revendication**

Procédé de modification de fines ou fibres de pâte de bois provenant d'un procédé de formation d'une pâte mécanique, consistant à faire réagir les fibres avec le N,N'-méthylène-bis-acrylamide dans un milieu alcalin aqueux ayant un pH compris entre 8,5 et 13,5 et ayant une consistance d'au moins 10%, à l'abri de l'air et en maintenant la température du milieu aqueux au dessous de son point d'ébullition.